Europäisches Patentamt

European Patent Office

Office européen des brevets

(11) Numéro de publication : **0 307 322 B1**

(12) **FASCICULE DE BREVET EUROPEEN**

(45) Date de publication du fascicule du brevet :
**18.03.92 Bulletin 92/12**

(51) Int. Cl.⁵ : **B29C 39/40, B29C 39/02,
B29C 39/00, B29D 31/00,
A61C 5/00**

(21) Numéro de dépôt : **88402286.4**

(22) Date de dépôt : **09.09.88**

(54) **Procédé de réalisation d'une pièce en résine par photopolymérisation et applications de ce procédé.**

(30) Priorité : **11.09.87 FR 8712645**

(43) Date de publication de la demande :
**15.03.89 Bulletin 89/11**

(45) Mention de la délivrance du brevet :
**18.03.92 Bulletin 92/12**

(84) Etats contractants désignés :
**AT BE CH DE ES FR GB GR IT LI LU NL SE**

(56) Documents cités :
**EP-A- 0 077 741
EP-A- 0 226 123
WO-A-86/05085**

(56) Documents cités :
**FR-A- 1 105 159
FR-A- 2 381 518
GB-A- 835 311
GB-A- 2 016 994
US-A- 3 254 411**

(73) Titulaire : **FRAMATOME
Tour Fiat 1, Place de la Coupole
F-92400 Courbevoie (FR)**

(72) Inventeur : **Puvilland, Gabriel
4 Place Saint-Antoine
F-78150 Le Chesnay (FR)**

(74) Mandataire : **Bouget, Lucien et al
Cabinet Lavoix 2, Place d'Estienne d'Orves
F-75441 Paris Cédex 09 (FR)**

## Description

L'invention concerne un procédé de réalisation d'une pièce en résine par photopolymérisation, avec un faible taux de retrait et des applications de ce procédé, en particulier dans le domaine de la restauration des dents et des prothèses dentaires ainsi que des prothèses chirurgicales.

Les résines plastiques composites dont on assure le durcissement par photopolymérisation sont utilisées de plus en plus comme matériaux de remplissage, de collage, de revêtement ou d'impression ou pour d'autres usages où leur facilité et leur rapidité de mise en oeuvre peuvent être un avantage décisif.

En particulier, dans le domaine de la dentisterie restauratrice et des prothèses dentaires, de telles résines photopolymérisables sont utilisées de plus en plus fréquemment, à la place de matériaux de type métallique.

De même, dans le domaine de la chirurgie osseuse, ces matériaux sont utilisés de plus en plus pour la restauration des os ou pour la fabrication de prothèses.

Ces résines plastiques composites sont généralement constituées de monomères ou d'un prépolymère mélangés à un diluant pour ajuster sa fluidité et à divers additifs favorisant la photopolymérisation. A ce mélange qu constitue la matrice de la résine composite, on ajoute un proportion variable suivant les caractéristiques désirées de charge constituée de grains ou fibres très fins de quartz, silice, etc ... et destinée à accroître les propriétés mécaniques (dureté, résistance) de la résine composite.

Dans toutes ces applications et en particulier lorsque la polymérisation doit être effectuée in situ, la souplesse et la rapidité de la photopolymérisation, c'est-à-dire de la polymérisation sous l'effet d'un rayonnement lumineux, constituent des avantages précieux et facilitent la tâche du praticien.

L'utilisation de sources lumineuses performantes en ce qui concerne la photopolymérisation et en particulier du laser à argon a permis d'accroître le champ d'application et les possibilités pratiques d'utilisation de ce procédé.

Cependant, la polymérisation des résines composites s'accompagne toujours d'un retrait volumique qui, dans de nombreuses applications, entraîne des inconvénients importants et limite les performances des résines polymérisables.

Il en est ainsi dans le cas de la restauration dentaire ou osseuse où le retrait de polymérisation peut faire apparaître des contraintes qui sont susceptibles d'induire des douleurs chez le patient ou de provoquer une certaine dégradation des prothèses telles que des fissurations ouvrant la voie à des infections bactériennes.

Dans le cas d'autres applications, et en particulier dans le cas de la fabrication ou de l'assemblage de pièces industrielles de grande précision, le retrait de polymérisation rend difficile le respect de cotes précises et provoque l'apparition de fissures ou de décollements, en particulier dans les zones de jonction entre les pièces.

On connaît par le GB-A-835.311, un procédé de réalisation d'une pièce en matière plastique consistant à former une ébauche d'une taille inférieure à la pièce à réaliser, par moulage et polymérisation de la matière plastique, puis à effectuer la polymérisation d'un volume complémentaire de matière plastique autour de l'ébauche, de manière à la faire adhérer à l'ébauche. On réduit ainsi le retrait de la pièce. Cependant, il n'est pas prévu de réaliser la polymérisation sous l'effet d'un rayonnement tel qu'un rayonnement lumineux.

Le FR-A-1.105.159 décrit un procédé de moulage de résines synthétiques dans lequel on réalise la polymérisation par couches successives, en partant de la ou des surfaces que la résine doit épouser exactement. Ce procédé ne permet pas d'obtenir la pièce de manière simple et rapide.

Dans le GB-A-2.016.994, on décrit un procédé de fabrication de prothèses dentaires par polymérisation d'une matière plastique sous l'effet d'un rayonnement. Le procédé qui comporte des étapes successives de polymérisation de différentes parties de la prothèse dentaire réalisées en une même matière plastique photopolymérisable est d'une mise en oeuvre délicate.

Le but de l'invention est donc de proposer un procédé de réalisation d'une pièce en résine, par photopolymérisation avec un faible taux de retrait, consistant à réaliser la pièce par mise en forme de matières à base de résine dans un état déformable non polymérisé, puis par polymérisation de la pièce, sous l'effet d'un rayonnement lumineux, la pièce étant constituée par la juxtaposition d'un volume principal au moins égal à 80 % du volume total de la pièce, d'une première matière à base de résine photopolymérisable et d'un volume complémentaire d'une seconde matière à base de résine photopolymérisable, à l'état non polymérisé, et la photopolymérisation du volume principal étant réalisée dans un premier temps et la photopolymérisation du volume complémentaire dans un second temps, le volume complémentaire étant maintenu en contact dans sa position d'assemblage, avec le volume principal, ce procédé étant d'une mise en oeuvre simple et rapide, en particulier dans le cas de la restauration dentaire.

Dans ce but, le volume principal est constitué par une matière photopolymérisable à une première longueur d'onde $\lambda 1$ et le volume complémentaire est constitué par une seconde matière photopolymérisable à une seconde longueur d'onde $\lambda 2$, mais insensible à la longueur d'onde $\lambda 1$, l'ensemble de la pièce étant exposé, dans un premier temps à un rayonnement de longueur d'onde $\lambda 1$, puis, dans un second temps, à un rayonnement de longueur d'onde λ2.

Afin de bien faire comprendre l'invention, on va maintenant décrire, à titre d'exemple non limitatif, en se référant aux figures jointes en annexe, un mode de mise en oeuvre du procédé suivant l'invention, dans le cas de la réparation d'une dent par réalisation d'une pièce de remplissage à l'intérieur d'une cavité dentaire.

La figure 1 est une vue en coupe de la cavité dentaire et de sa pièce de remplissage, avant photopolymérisation.

La figure 2 est une vue en coupe analogue à la figure 1 de la cavité dentaire après photopolymérisation.

Les figures 3, 4 et 5 sont des vues en coupe de la cavité dentaire au cours de trois étapes successives du procédé de réalisation de la pièce de remplissage effectué suivant une variante de réalisation.

La figure 6 est une vue en coupe de la cavité dentaire, pendant la phase finale de mise en oeuvre du procédé suivant l'invention et suivant une seconde variante.

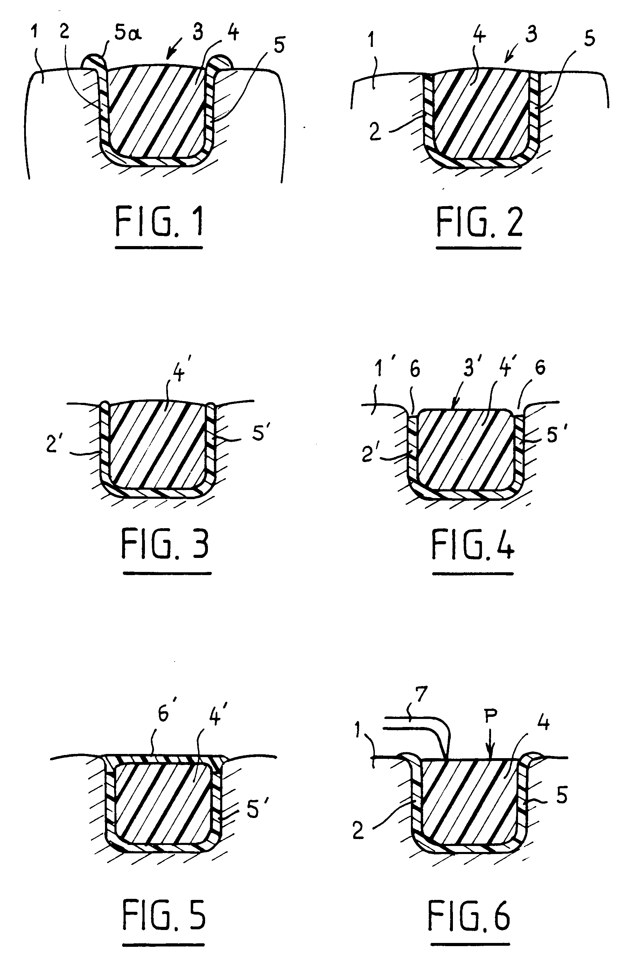

Sur la figure 1, on voit une dent 1 présentant une cavité 2 dont on veut effectuer le remplissage par une pièce en résine dont le moulage et la polymérisation sont réalisés directement dans la cavité 2.

La cavité 2 est nettoyée et meulée à la fraise de manière habituelle, avant la mise en place de la pièce 3 sous une forme non polymérisée et, en conséquence, relativement fluide et déformable.

La consistance du mélange photopolymérisable est généralement celle d'une pâte très souple.

Selon l'invention, la pièce de remplissage 3 est constituée par juxtaposition d'un volume principal 4 d'une première matière photopolymérisable constituée par une mélange et d'un volume complémentaire 5 d'une seconde matière photopolymérisable constituée par un mélange différent du mélange constituant le volume principal 4.

De façon préférentielle et comme représenté sur la figure 1, le volume complémentaire 5 constitue une couche continue de faible épaisseur recouvrant l'ensemble de la paroi de la cavité 2, le volume principal 4 constituant la partie centrale et la surface extérieure de la pièce de remplissage 3.

Le volume principal 4 de la pièce de remplissage 3 représente sensiblement 90 % du volume total de la pièce définitive telle que représentée sur la figure 2.

Le volume complémentaire 5 représente un peu plus de 10 % du volume total de la pièce 3 et, par exemple, sensiblement 11 % de ce volume total. La matière en excès du volume complémentaire 5 constitue un bourrelet de réserve 5a sur le bord de la cavité 2.

Dans un premier mode de réalisation du procédé suivant l'invention, on reconstitue le volume principal 4, de la pièce 3 avec une première matière photopolymérisable constituée par un mélange sensibilisé de façon que sa photopolymérisation ait lieu lorsque ce mélange est exposé à un rayonnement laser ayant une première longueur d'onde λ1.

Le volume complémentaire 5 est constitué par un mélange photopolymérisable insensible au rayonnement laser de longueur d'onde λ1 et dont la photopolymérisation a lieu sous l'effet d'un rayonnement laser de longueur d'onde λ2.

Après remplissage de la cavité 2 par la pièce 3 constituée comme décrit prcédemment, on expose le volume principal 4 de la pièce 3 à un rayonnement laser de longueur d'onde λ1, par exemple d'un rayonnement provenant d'un laser à argon. Ce rayonnement produit la polymérisation du volume principal 4 qui reste en contact par sa surface extérieure avec le volume complémentaire externe 5. La fluidité de la matière non polymérisée constituant le volume complémentaire 5 est telle que cette matière assure la sustentation du volume principal 4 tout en restant constamment en contact intime par toute sa surface avec la surface extérieure de ce volume 4.

Pendant son durcissement par photopolymérisation et après durcissement, le volume principal 4 se trouve dans la situation d'un corps libre dans une masse visqueuse.

Le retrait de polymérisation du corps principal 4 est donc automatiquement compensé par la matière du volume complémentaire 5 dont la fluidité est suffisante pour que cette matière suive les déplacements de la surface de la pièce 4 au cours de son retrait et remplisse toute cavité de très faible volume qui pourrait être créée par ce retrait.

Lorsque la polymérisation et le retrait du volume principal 4 ont été réalisés, la majeure partie de la réserve de matière 5a qui a servi à la compensation du retrait, a pénétré à l'intérieur de la cavité 2.

On expose alors le volume complémentaire 5 de matière polymérisable au rayonnement laser de longueur d'onde λ2, pour obtenir la photopolymérisation de ce volume complémentaire et le collage de l'ensemble du remplissage sur la paroi de la cavité, le volume complémentaire étant en contact avec le volume prncipal dans sa position d'assemblage et avec la paroi de la cavité 2 de la dent 1.

On peut améliorer l'adhérence du volume complémentaire sur la paroi de la cavité en réalisant un mordançage de cette paroi préalablement au remplissage de la cavité par la pièce à l'état non polymérisé.

Le retrait du volume complémentaire 5 est très faible en valeur absolue, le volume 5 représentant lui-même une fraction faible du volume total de la pièce 3.

Il en résulte une liaison parfaite entre la pièce de remplissage 3 et les parois de la cavité 2 en même temps que des contraintes pratiquement nulles que ce soit dans la dent, dans la pièce de remplissage ou à l'interface des deux.

Il est possible d'utiliser une quantité de matière de réserve 5a sensiblement supérieure au retrait prévisible du volume principal 4 de la pièce 3 et, dans ce cas, la matière excédentaire de la partie 5 de la pièce 3 sera supprimée et arasée à la fraise après durcissement du volume 5.

Il est possible de prévoir un volume complémentaire dont la photopolymérisation sera assurée dans un deuxième temps, représentant plus de 10 % du volume total de la pièce.;

Cependant, ce volume complémentaire ne devra pas être trop important pour que le retrait final non compensé de ce volume complémentaire reste dans des limites acceptables.

C'est la raison pour laquelle le volume principal est choisi de façon à représenter au moins 80 % du volume total de la pièce.

Le retrait linéaire des résines habituellement utilisées en dentisterie telles que les résines indiquées plus loin étant de 0,2 à 1 %, on limite ainsi dans tous les cas le retrait final non compensé de la pièce à une valeur inférieure au retrait qui devait être accepté dans un procédé selon l'art antérieur et dans le meilleur des cas.

Pour réaliser le remplissage d'une cavité dentaire par le procédé qui vient d'être décrit, on a utilisé pour constituer le volume principal 4 de la pièce de remplissage, la composition donnée ci-dessous. Pour la matrice :

– 3,20 % de Benzoine Methyl Ether (amorceur)
– 1,75 % de Methyldiethanol amine (accélérateur)
– 0,05 % d'Eosine (sensibilisateur)
– 95 % de résine de base constituée d'un mélange de monomère BIS-GMA (Bisphénol A bis (2 - hydroxypropyl) méthacrylate) et de diluant TEGDMA (Triethylène Glycol Diméthacrylate) en parts égales. La charge était constituée de particules de quartz de 200 μm et représentait 80 % en poids de la résine composite.

Les pourcentages donnés ci-dessus étant des pourcentages pondéraux.

L'utilisation simultanée d'un amorceur et d'éosine dans le mélange permet d'obtenir une photopolymérisation rapide du mélange photopolymérisable, lorsque celui-ci est exposé à un rayonnement provenant d'un laser à argon accordé sur une longueur d'onde de 514 nm.

Pour réaliser le volume complémentaire 5 de la pièce de remplissage, on a utilisé la composition suivante. Pour la matrice :

– 0,3 % de Camphoroquinone (photo-amorceur)
– 1,0 % de Méthyldiethanolamine (accélérateur)
– 98,7 % de Résine de base chargée de composition identique à celle indiquée pour le volume principal.

Pour la charge : nature et proportion identiques à celles du volume principal.

Les pourcentages indiqués ci-dessus étant des pourcentages pondéraux.

En utilisant la Camphoroquinone comme agent photo-amorceur, on obtient un mélange photopolymérisable dont la polymérisation est assurée par exposition au rayonnement d'un laser à argon accordé sur une longueur d'onde de 488 nm.

On a utilisé comme source d'irradiation des résines photopolymérisables, un laser à argon monochrome qui est accordé successivement, pour la mise en oeuvre du procédé, sur les longueurs d'onde 514 nm et 488 nm.

Dans chacun des cas, les longueurs d'onde les plus intenses situées au voisinage de 514 nm et 488 nm respectivement assureront la photopolymérisation du mélange photopolymérisable correspondant constituant le volume principal ou le volume complémentaire de la pièce de remplissage.

A la place des additifs qui ont été indiqués ci-dessus et qui permettent la photopolymérisation du mélange aux longueurs d'ondes indiquées, il est possible d'utiliser d'autres additifs tels que :

– comme amorceur :
un Alkyléther de Benzoïne
un dérivé d'acétophénone
la Benzoïne oxime
la Benzophénone
– comme sensibilisateur :
l'EthylEoisine à 532 nm
l'Erythrosine à 525 nm
le Bleu de Méthylène à 632 nm
– comme photoamorceur, à 488 nm :
la parabenzoquinone ou toute dicétone
– comme accélérateur (toutes longueurs d'onde) :
le méthyldiethanolamine
le méthyléthanoldiamine
le N, N dimétylaminoéthylméthacrylate
les amines tertiaires

La substance utilisée comme amorceur représente dans tous les cas de 0 à 15 % de la masse totale de la résine avant incorporation de la charge (matrice), le sensibilisateur de 0 à 1 %, le photoamorceur de 0 à 10 % et l'accélérateur de 0 à 15 %.

De même, au lieu des résines composites indiquées ci-dessus, on peut utiliser :

– comme monomère ou prépolymère constituant la matrice organique, toutes les résines méthacrylates mono ou poly-fonctionnelles notamment :
le Métacrylate de méthyle (MMA)
le Bisphénol A diméthacrylate
l'Ethylène glycol diméthacrylate
le Trimethylol Propane Triméthacrylate (TMP-TMA)
l'Uréthane diméthacrylate (UEDMA)
– comme charge associée renforçant les propriétés mécaniques : des grains de 0,01 à 200 $\mu$m ou fibres de 200 $\mu$m à 5 mm de quartz, silice, verres, polymères, broyés ou synthétisés en granulométrie unique ou mélangée, enrobés, avant incorporation dans la matrice et pour améliorer la liaison avec elle, d'un produit de couplage à molécules dipolaires telles que les organosilanes.

Il est également possible d'effectuer les deux photopolymérisations successives mises en oeuvre dans le procédé selon l'invention, à une seule longueur d'onde. Dans ce cas, on ajoutera à l'un des mélanges photopolymérisables à base de résine, un retardateur de polymérisation constitué par exemple par les dérivés de l'hydroquinone tel que l'hydroquinone diméthyléther, ou par les dérivés du phénol tels que l'Aminophénol.

La substance utilisée comme retardateur représente dans tous les cas de 0,01 à 0,10 % en poids de la résine avant incorporation de la charge (matrice).

Ce mélange photopolymérisable contenant un retardateur est destiné à constituer le volume complémentaire de la pièce.

Après remplissage de la cavité par la pièce de remplissage constituée par la juxtaposition du mélange de résine sans retardateur et avec retardateur de polymérisation, on expose la pièce de remplissage à un rayonnement d'une longueur d'onde adéquate. La photopolymérisation du mélange constituant le volume principal 4 aura lieu, dans un premier temps, et la photopolymérisation de la résine additionnée de retardateur constituant le volume complémentaire, dans un second temps.

Pendant la polymérisation du volume principal de la pièce, le retrait de ce volume pourra être compensé par le mélange fluide constituant le volume complémentaire non polymérisé.

Sur les figures 3, 4 et 5, on voit une variante de réalisation du procédé, le volume complémentaire 5' occupant à l'instant initial (figure 3) exactement le volume restant dans lacavité 2', autour du volume principal 4'.

Après photopolymérisation du volume principal 4' (figure 4), la résine du volume 5' qui a servi à la compensation du retrait du volume principal 4' a pénétré dans la cavité 2' si bien qu'après photopolymérisation du volume complémentaire 5', le retrait de ce volume s'est accompagné de l'apparition d'une partie en creux 6 de la pièce 3' par rapport à la dent 1'.

Cette partie en creux 6 peut être remplie par une matière de rebouchage 6' déposée sur la pièce 3' après son durcissement par polymérisation. La couche 6' peut être également constituée par une résine photopolymérisable dont on assure le durcissement par exposition à un rayonnement laser.

Sur la figure 6, on a représenté une variante de mise en oeuvre du procédé, la mise en contact du volume principal 4 de la pièce de remplissage 3 avec le volume complémentaire 5, dans leur position d'assemblage à l'intérieur de la cavité 2, pendant la photopolymérisation du volume complémentaire 5, étant assurée par une pression P exercée grâce à un outil 7 mis en appui sur le volume principal 4 de la pièce 3, après son durcissement par polymérisation.

Il est également possible de réaliser la photopolymérisation du volume principal de remplissage de la cavité avant de mettre en place le volume complémentaire de la pièce constituant la couche de collage et de compensation du retrait de polymérisation. Pour celà, on prépare la paroi de la cavité 2 avant remplissage de façon à éviter le collage du volume principal lors de sa polymérisation. On peut par exemple appliquer une substance adéquate telle qu'une huile sur la paroi. On effectue ensuite le remplissage total de la cavité par une matière photopolymérisable constituant le volume principal de la pièce de réparation. Le durcissement du volume principal est effectué in situ par photopolymérisation comme décrit ci-dessus. Le volume principal est ensuite facilement extrait de la cavité grâce à son retrait et à la présence d'un produit huileux évitant le collage sur la paroi de la cavité. Pour cela, on a évidemment pris soin avant remplissage de façonner la cavité pour éviter tout blocage mécanique de la pièce. La paroi de la cavité est ensuite nettoyée et éventuellement mordancée. Un volume complémentaire de matière photopolymérisable est appliqué sur la paroi et le volume principal est mis en place en contact d'assemblage avec le volume complémentaire. On réalise alors la photopolymérisation du volume complémentaire qui assure à la fois la compensation du retrait et le collage du volume principal.

Le procédé suivant l'invention peut s'appliquer non seulement au cas de la restauration de dents grâce à des pièces de remplissage mises en place et photopolymérisées dans des cavités dentaires mais encore à la fabrication de prothèses dentaires, à l'intérieur d'empreintes ou de moules.

Dans ce cas, l'empreinte ou le moule jouera le rôle de la cavité dentaire dont on assure le remplissage, comme il a été décrit ci-dessus.

Le procédé suivant l'invention peut également être appliqué dans le cadre de la chirurgie réparatrice, pour la reconstitution partielle de certains os.

Le procédé peut également être utilisé pour la réalisation de prothèses osseuses complètes.

Le procédé suivant l'invention peut également connaître de nombreuses applications dans le cadre de la fabrication de pièces en résine plastique qui doivent être obtenues avec une très grande précision de forme et de dimension. Dans ce cas, la compensation de la plus grande partie du retrait de polymérisation, pendant le moulage et le durcissement de la pièce, permet d'obtenir une très grande précision dimensionnelle. Le procédé suivant l'invention est donc susceptible de nombreuses applications dans des industries variées utilisant des pièces en résine et, en particulier, dans le domaine de l'électronique.

## Revendications

1. Procédé de réalisation d'une pièce (3) en résine, par photopolymérisation avec un faible taux de retrait, consistant à réaliser la pièce (3) par mise en forme de matières à base de résine dans un état déformable non polymérisé, puis par polymérisation de la pièce (3), sous l'effet d'un rayonnement lumineux, la pièce (3) étant constituée par la juxtaposition d'un volume principal (4) au moins égal à 80 % du volume total de la pièce (3), d'une première matière à base de résine photopolymérisable et d'un volume complémentaire (5) d'une seconde matière à base de résine photopolymérisable, à l'état non polymérisé, et la photopolymérisation du volume principal (4) étant réalisée dans un premier temps et la photopolymérisation du volume complémentaire dans un second temps, le volume complémentaire (5) étant maintenu en contact dans sa position d'assemblage, avec le volume principal (4), caractérisé par le fait que le volume principal (4) est constitué par une matière photopolymérisable à une première longueur d'onde ($\lambda$1) et que le volume complémentaire (5) est constitué par une seconde matière photopolymérisable à une seconde longueur d'onde ($\lambda$2), mais insensible à la longueur d'onde ($\lambda$1), l'ensemble de la pièce (3) étant exposé, dans un premier temps à un rayonnement de longueur d'onde ($\lambda$1), puis, dans un second temps, à un rayonnement de longueur d'onde ($\lambda$2).

2. Procédé suivant la revendication 1, caractérisé par le fait que les matières photopolymérisables sont sous la forme d'un matériau composite comportant une résine de base constituée par un mélange de monomères ou prépolymères, un diluant pour ajuster la viscosité, une charge de renforcement et des additifs de photopolymérisation.

3. Procédé suivant la revendication 2, caractérisé par le fait que la résine (monomères ou prépolymères) est une résine méthacrylate mono ou polyfonctionnelle.

4. Procédé suivant la revendication 3, caractérisé par le fait que la résine monomère ou prépolymère est constituée par l'une au moins des substances suivantes :
le BIS-GMA
le Métacrylate de méthyle (MMA)
le Bisphénol A diméthacrylate
l'Ethylène glycol diméthacrylate
le Triméthylol Propane (TMPTMA)
l'Uréthane diméthacrylate (UEDMA).

5. Procédé suivant l'une quelconque des revendications 2 à 4, caractérisé par le fait que la charge de renforcement de la résine est constituée par l'une au moins des substances suivantes : quartz, silice, verres ou polymères sous forme de grains de 0,01 à 200 $\mu$m ou sous forme de fibres de 200 $\mu$m à 5 mm de longueur.

6. Procédé suivant l'une quelconque des revendications 2 à 5, caractérisé par le fait que les additifs de photopolymérisation comportent au moins un amorceur, au moins un sensibilisateur et au moins un accélérateur dans des proportions au plus égales à 15 %, 1 % et 15 % respectivement de la masse totale de matière photopolymérisable.

7. Procédé suivant la revendication 6, caractérisé par le fait que l'amorceur est constitué par l'une des substances suivantes :
le Benzoïne Méthyl Ether
un Alkyléther de Benzoïne
un dérivé d'acétophénone
la Benzophénone,
et que le sensibilisateur est constitué par l'une des substances suivantes :
l'Eosine
l'Ethyléosine
l'Erythrosine
le bleu de Méthylène.

8. Procédé suivant l'une quelconque des revendications 2 à 5, caractérisé par le fait que les additifs de photopolymérisation comportent au moins un photoamorceur et un accélérateur dans des proportions respectives au plus égale sà 10 % et 15 % de la masse totale de matière photopolymérisable.

9. Procédé suivant la revendication 8, caractérisé par le fait que le photoamorceur est constitué par une au moins des subhstances suivantes :
la camphoroquinone
la parabenzoquinone
une dicétone.

10. Procédé suivant l'une quelconque des revendications 6 à 9, caractérisé par le fait que l'accélérateur est constitué par au moins une des substances suivantes :
le méthyldiethanolamine
le méthyléthanoldiamine
le N, N diméthylaminoéthylméthacrylate
une amine tertiaire.

11. Procédé suivant la revendication 1, caractérisé par le fait que la photopolymérisation du volume principal (4) est réalisée avec un premier laser et la photopolymérisation du volume complémentaire (5) avec un second laser.

12. Procédé suivant la revendication 1, caractérisé par le fait que la photopolymérisation du volume principal (4) et la photopolymérisation du volume complémentaire (5) sont réalisées avec un laser ajustable sur deux longueurs d'onde différentes.

13. Procédé suivant la revendication 12, caractérisé par le fait que le laser est un laser à argon ajustable sur deux longueurs d'ondes principales à 488 et 514 nm.

14. Application du procédé suivant l'une quelconque des revendications 1 à 13, au cas de la restauration d'une dent (1) présentant une cavité dentaire (2) caractérisée par le fait que la pièce en résine est une pièce de remplissage (3) de la cavité (2) réalisée par moulage et photopolymérisation, la photopolymérisation du volume complémentaire (5) assurant de plus le collage de l'ensemble de la pièce à la dent (1).

15. Application du procédé selon l'une des revendications 1 à 13 au cas de la restauration d'une dent (1) présentant une cavité dentaire (2), caractérisée par le fait que la pièce (3) est une pièce de remplissage de la cavité (2) réalisée par moulage in situ dans la cavité d'une pièce comportant un volume principal (4) et un volume complémentaire (5) à l'état non polymérisé puis par polymérisation in situ du volume principal et du volume complémentaire.

16. Application du procédé selon la revendication 15, caractérisée par le fait que le volume complémentaire (5) de la pièce (3) à l'état non polymérisé constitue une réserve (5a) débordante par rapport à la surface de la dent (1) pour compenser le retrait du volume principal (4) de la pièce (3), lors de sa polymérisation.

17. Application suivant la revendication 15, caractérisée par le fait qu'après polymérisation du volume complémentaire (5) de la pièce de remplissage (3), on effectue le rebouchage (6') d'une partie en creux (6) à la surface de la dent (1) due au retrait du volume principal (4') et du volume complémentaire (5'), au cours de leur polymérisation.

18. Application suivant la revendication 15, caractérisée par le fait que pendant la photopolymérisation du volume complémentaire (5), on applique une pression P sur la pièce de remplissage (3), vers l'intérieur de la cavité (2) par appui sur le volume principal (4) photopolymérisé.

19. Application du procédé suivant l'une quelconque des revendications 1 à 13, au cas de la chirurgie réparatrice osseuse.

20. Application du procédé suivant l'une quelconque des revendications 1 à 13, dans le cas de la fabrication par moulage de pièces en résine de grande précision dimensionnelle.

21. Application suivant la revendication 20, dans le cas de pièces destinées à des appareillages électroniques.

**Patentansprüche**

1. Verfahren zur Herstellung eines Harzgegenstandes (3) durch Photopolymerisation mit geringem Schwund, das darin besteht, den Gegenstand (3) durch Formen von Materialien auf Harzbasis in einem deformierbaren, nichtpolymerisierten Zustand herzustellen und anschließend den Gegenstand (3) unter der Einwirkung von Lichtstrahlen zu polymerisieren, wobei der Gegenstand (3) aus der Anlagerung eines Hauptvolumens (4) aus einem ersten Material auf der Basis von photopolymerisierbarem Harz, das mindestens 80 % des Gesamtvolumens des Gegenstandes (3) ausmacht, und einem Komplementärvolumen (5) aus einem zweiten Material auf der Basis von photopolymerisierbarem Harz im nichtpolymerisierten Zustand resultiert und wobei die Photopolymerisation des Hauptvolumens (4) innerhalb eines ersten Zeitabschnittes und die Photopolymerisation des Komplementärvolumens (5) innerhalb eines zweiten Zeitabschnittes durchgeführt werden, wobei das Komplementärvolumen (5) mit dem Hauptvolumen (4) in seiner Anordnung zur Zusammenfügung in Kontakt bleibt, **dadurch gekennzeichnet**, daß das Hauptvolumen (4) aus einem bei einer ersten Wellenlänge ($\lambda$1) photopolymerisierbaren Material und daß das Komplementärvolumen (5) aus einem bei einer zweiten Wellenlänge ($\lambda$2) photopolymerisierbaren, aber gegen die Wellenlänge ($\lambda$1) unempfindlichen Material bestehen, und daß der gesamte Gegenstand (3) in einem ersten Zeitabschnitt einer Strahlung mit der Wellenlänge ($\lambda$1) und dann in einem zweiten Zeitabschnitt einer Strahlung mit der Wellenlänge ($\lambda$2) ausgesetzt wird.

2. Verfahren nach Anspruch 1, dadurch gekennzeichnet, daß die photopolymerisierbaren Materialien in Form eines Verbundmaterials vorliegen, das ein Basisharz aus einem Gemisch von Monomeren oder Präpolymeren, einen Verdünner zur Einstellung der Viskosität, einen Füllstoff zur Verstärkung und Additive zur Photopolymerisation enthält.

3. Verfahren nach Anspruch 2, dadurch gekennzeichnet, daß das Harz (Monomere oder Präpolymere) ein mono- oder polyfunktionelles Methacrylatharz ist.

4. Verfahren nach Anspruch 3, dadurch gekennzeichnet, daß das Harz aus Monomeren oder Präpolymeren aus mindestens einer der folgenden Substanzen besteht:
BIS-GMA
Methylmethacrylat (MMA)
Bisphenol A-Dimethacrylat
Ethylenglycoldimethacrylat
Trimethylolpropantrimethacrylat (TMPTMA)
Harnstoffdimethacrylat (UEDMA).

5. Verfahren nach einem der Ansprüche 2 bis 4, dadurch gekennzeichnet, daß der Füllstoff zur Verstärkung des Harzes aus mindestens einer der folgenden Substanzen besteht: Quarz, Kieselsäure, Gläser oder Polymere in Form von Körnern von 0,01 bis 200 $\mu$m oder in Form von Fasern einer Länge von 200 $\mu$m bis 5 mm.

6. Verfahren nach einem der Ansprüche 2 bis 5, dadurch gekennzeichnet, daß die Additive zur Photopolymerisation mindestens einen Initiator, mindestens einen Sensibilisator und mindestens einen Beschleuniger in Anteilen von maximal 15 %, 1 % bzw. 15 %, bezogen auf die Gesamtmasse photopolymerisierbaren Materials, enthalten.

7. Verfahren nach Anspruch 6, dadurch gekennzeichnet, daß der Initiator aus einer der folgenden Substanzen besteht:
Benzoinmethylether
einem Benzoinalkylether
einem Acetophenonderivat
Benzophenon,
und daß der Sensibilisator aus einer der folgenden Substanzen besteht:
Eosin
Ethyleosin
Erythrosin
Methylenblau.

8. Verfahren nach einem der Ansprüche 2 bis 5, dadurch gekennzeichnet, daß die Additive zur Photopolymerisation mindestens einen Photoinitiator und einen Beschleuniger in Anteilen von maximal 10 % und 15 %, bezogen auf die Gesamtmasse photopolymerisierbaren Materials, enthalten.

9. Verfahren nach Anspruch 8, dadurch gekennzeichnet, daß der Photoinitiator aus mindestens einer der folgenden Substanzen besteht:
Campherchinon
Parabenzochinon
einem Diketon.

10. Verfahren nach einem der Ansprüche 6 bis 9, dadurch gekennzeichnet, daß der Beschleuniger aus mindestens einer der folgenden Substanzen besteht:
Methyldiethanolamin
Methylethanoldiamin
N,N-Dimethylaminoethylmethacrylat
einem tertiären Amin.

11. Verfahren nach Anspruch 1, dadurch gekennzeichnet, daß die Photopolymerisation des Hauptvolumens (4) mit einem ersten Laser und die Photopolymerisation des Komplementärvolumens (5) mit einem zweiten Laser durchgeführt werden.

12. Verfahren nach Anspruch 1, dadurch gekennzeichnet, daß die Photopolymerisation des Hauptvolumens (4) und die Photopolymerisation des Komplementärvolumens (5) mit einem auf zwei verschiedene Wellenlängen einstellbaren Laser durchgeführt werden.

13. Verfahren nach Anspruch 12, dadurch gekennzeichnet, daß der Laser ein Argonlaser ist, der auf zwei Hauptwellenlängen von 488 und 514 nm einstellbar ist.

14. Anwendung des Verfahrens nach einem der Ansprüche 1 bis 13 für den Fall der Wiederherstellung eines Zahns (1) mit einem Loch (2), dadurch gekennzeichnet, daß der Harzgegenstand ein Gegenstand zum Auffüllen (3) des Loches (2), hergestellt durch Formen und Photopolymerisation, ist, wobei die Photopolymerisation des Komplementärvolumens (5) außerdem das Haften des Gesamtgegenstandes am Zahn (1) sichert.

15. Anwendung des Verfahrens nach einem der Ansprüche 1 bis 13 für den Fall der Wiederherstellung eines Zahns (1) mit einem Loch (2), dadurch gekennzeichnet, daß der Gegenstand (3) ein Gegenstand zum Auffüllen des Loches (2) ist, der durch in-situ-Formen eines Gegenstandes im Loch, der ein Hauptvolumen (4) und ein Komplementärvolumen (5) im nichtpolymerisierten Zustand enthält, und anschließendes in-situ-Polymerisieren des Hauptvolumens und des Komplementärvolumens hergestellt wird.

16. Anwendung des Verfahrens nach Anspruch 15, dadurch gekennzeichnet, daß das Komplementärvolumen (5) des Gegenstandes (3) im nichtpolymerisierten Zustand einen Überschuß (5a) darstellt, der über die Oberfläche des Zahns (1) ragt, um den Schwund des Hauptvolumens (4) des Gegenstandes (3) während der Polymerisation zu kompensieren.

17. Anwendung nach Anspruch 15, dadurch gekennzeichnet, daß man nach der Polymerisation des Komplementärvolumens (5) des Gegenstandes zum Auffüllen (3) auf der Oberfläche des Zahns (1) die Abdichtung (6') eines vertieften Bereiches (6) aufbringt, der aufgrund des Schwundes des Hauptvolumens (4') und des Komplementärvolumens (5') im Verlauf ihrer Polymerisation auftritt.

18. Anwendung nach Anspruch 15, dadurch gekennzeichnet, daß man während der Photopolymerisation des Komplementärvolumens (5) einen Druck P auf den Gegenstand zum Auffüllen (3) gegen das Innere des Loches (2) durch Abstützen des photopolymerisierten Hauptvolumens (4) ausübt.

19. Anwendung des Verfahrens nach einem der Ansprüche 1 bis 13 für den Fall der wiederherstellenden Knochenchirurgie.

20. Anwendung des Verfahrens nach einem der Ansprüche 1 bis 13 für den Fall der Herstellung von Harzgegenständen von großer Dimensionsgenauigkeit durch Formen.

21. Anwendung nach Anspruch 20 für den Fall von Gegenständen für elektronische Apparaturen.

## Claims

1. Process for producing a component (3) made of resin, by photopolymerization with a low degree of shrinkage, consisting in producing the component (3) by forming resin-bases material in an unpolymerized deformable state and then polymerizing the component (3) under the effect of a luminous radiation, the component (3) being formed by juxtaposition of a main volume (4) equal to at least 80% of the total volume of the component (3), of a resin-based first photopolymerizable material and of a complementary volume (5) of a resin-based second photopolymerizable material, in an unpolymerized state, the photopolymerization of the main volume (4) being carried out in a first step and the photopolymerization of the complementary volume being carried out in a second step, the main volume (4) and the complementary volume (5) being maintained in contact in their assembly position, characterised in that the main volume (4) consists of a material which is photopolymerizable at a first wavelength (λ1), and that the complementary volume (5) consists of a second material which is photopolymerizable at a second wavelength (λ2), but insensitive to the wavelength (λ1), the whole component (3) being exposed, in a first step, to a radiation of wavelength (λ1) and then, in a second step, to a radiation of wavelength (λ2).

2. Process according to Claim 1, characterised in that the photopolymerizable materials are in the form of a composite material comprising a base resin mixture of monomers or prepolymers, a diluent for adjusting the viscosity, a reinforcing filler and photopolymerization additives.

3. Process according to Claim 2, characterised in that the resin (monomers or prepolymers) is a mono- or polyfunctional methacrylate resin.

4. Process according to Claim 3, characterised in that the monomer or prepolymer resin consists of at least one of the following substances:
BIS-GMA
methyl methacrylate (MMA)
bisphenol A dimethacrylate
ethylene glycol dimethacrylate
trimethylolpropane (TMPTMA)
urethane dimethacrylate (UEDMA).

5. Process according to any one of Claims 2 to 4, characterised in that the filler for reinforcing the resin consists of at least one of the following substances: quartz, silica, glasses or polymers in the form of particles from 0.01 to 200 μm or in the form of fibres from 200 μm to 5 mm in length.

6. Process according to any one of Claims 2 to 5, characterised in that the photopolymerization additives comprise at least one initiator, at least one sensitizer and at least one accelerator, in proportions not exceeding 15%, 1% and 15% respectively of the total mass of photopolymerizable material.

7. Process according to Claim 6, characterised in that the initiator consists of one of the following substances:
benzoin methyl ether
a benzoin alkyl ether
an acetophenone derivative
benzophenone,
and that the sensitized consists of one of the following substances:
eosin
ethyleosin
erythrosin
methylene blue.

8. Process according to one of Claims 2 to 5, characterised in that the photopolymerization additives comprise at least one photoinitiator and an accelerator in respective proportions not exceeding 10% and 15% of the total mass of photopolymerizable material.

9. Process according to Claim 8, characterised in that the photoinitiator consists of at least one of the following substances:
camporoquinone
para-benzoquinone
a diketone.

10. Process according to any one of Claims 6 to 9, characterised in that the accelerator consists of at least one of the following susbtances:
methyldiethanolamine
methylethanoldiamine
N,N-dimethylaminoethyl methacrylate
a tertiary amine.

11. Process according to Claim 1, characterised in that the photopolymerization of the main volume (4) is carried out with a first laser and the photopolymerization of the complementary volume (5) with a second laser.

12. Process according to Claim 1, characterised in that the photopolymerization of the main volume (4) and the photopolymerization of the complementary volume (5) are carried out with a laser which can be adjusted to two different wavelengths.

13. Process according to Claim 12, characterised in that the laser is an argon laser adjustable to two principal wavelengths at 488 and 514 nm.

14. Application of the process according to any one of Claims 1 to 13, to the case of the restoration of a tooth (1) exhibiting a dental cavity (2), characterised in that the resin component is a component for filling (3) the cavity (2) produced by moulding and photopolymerization, the photopolymerization of the complementary volume (5) additionally ensuring the adhesive bonding of the whole component to the tooth (1).

15. Application of the process according to one of Claims 1 to 13 to the case of the restoration of a tooth (1) exhibiting a dental cavity (2), characterised in that the component (3) is a component for filling the cavity (2), produced by moulding in situ in the cavity a component comprising a main volume (4) and a complementary volume (5) in the unpolymerized state and then by in situ polymerization of the main volume and of the complemetary volume.

16. Application of the process according to Claim 15, characterised in that the complementary volume (5) of the component (3) in the unpolymerized state forms a reserve (5a) overflowing in relation to the surface of the tooth (1) to compensate for the shrinkage of the main volume (4) of the component (3), while it is polymerizing.

17. Application according to Claim 15, characterised in that after the polymerization of the complementary volume (5) of the filling component (3) the stopping (6') of a hollow part (6) at the surface of the tooth (1), due to the shrinkage of the main volume (4')

and of the complementary volume (5') during their polymerization, is carried out.

18. Application according to Claim 15, characterised in that during the polymerization of the complementary volume (5) a pressure P is applied to the filling component (3) towards the interior of the cavity (2) by pressing on the photopolymerized main volume (4).

19. Application of the process according to any one of Claims 1 to 13 to the case of bone repair surgery.

20. Application of the process according to any one of Claims 1 to 13, in the case of the manufacture by moulding of resin components of high dimensional accuracy.

21. Application according to Claim 20, in the case of components intended for electronic apparatus.

FIG. 1

FIG. 2

FIG. 3

FIG. 4

FIG. 5

FIG. 6